# EUROPEAN PATENT APPLICATION

(11) **EP 1 903 051 A2**
(43) Date of publication of application: **26.03.2008**
(21) Application number: 07018400.7
(22) Date of filing: 19.09.2007
(51) Int. Cl.: C07J 53/00

(54) **Epoxidation of 17-oxo-15,16-methylene steroids with sulfoxonium ylides**

(30) Priority: 22.09.2006 IT MI20061802
(71) Applicant: Antibioticos S.p.A., 20090 Rodano (IT)
(72) Inventor: Cabri, Walter, 20089 Rozzano (MI) (IT); Benedetti, Fabio, 20052 Monza (MI) (IT); Alpegiani, Marco, 20132 Milano (IT); Rodriguez, Manuela, 80100 Napoli (IT); Botta, Cinzia, 85030 Castelnuovo di Sant`Andrea (IT)
(74) Representative: Minoja, Fabrizio

(57) **Abstract**

A process for the epoxidation of 17-oxo-15,16-methylene steroids, in particular of drospirenone precursors, comprising the use of sulfoxonium ylides, in particular of dimethylsulfoxonium methyl ylide. The process allows to prepare in good yields 17-spiro epoxides, which can be easily transformed into 17-spironolacto-steroids.

## Description

### Field of the invention

The present invention relates to a process for the epoxidation of 17-oxo-15,16-methylene steroids, in particular of drospirenone precursors.

### Background of the invention

Drospirenone (I) is a synthetic steroid with progestin, antimineralocorticoid and antiandrogen activity, used as contraceptive.

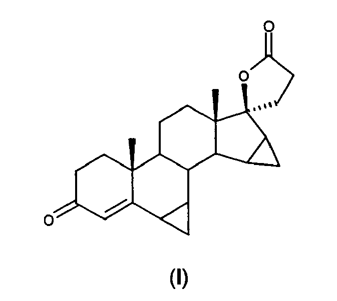

The synthesis of this compound comprises the conversion of a steroid precursor containing a C₁₇ keto group to the corresponding spironolactone. This conversion is usually carried out with carbanions, such as propargyl alcohol derivatives, as disclosed in US 6,121,465, US 2005/0192450 and EP 0075189. US 4,129,564 discloses the preparation of 17-spironolacto-steroids comprising the use of lithium and 1-bromo-3-dimethoxy-propane. J. Med. Chem. 1987, Vol. 30, n. 9 discloses the preparation of 15,16-methylene-17-spironolacto-methylene steroids comprising the treatment of a 15,16-methylene-17-keto-steroid in dimethylsulfoxonium methyl ylide; however, the desired product forms with low yields.

### Disclosure of the invention

The present invention relates to a process for the epoxidation of 17-oxo-15,16-methylene-steroids, in particular of drospirenone precursors (I) 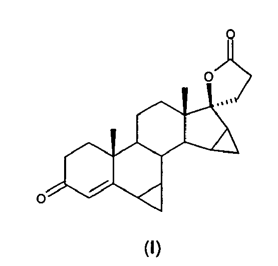 which comprises the treatment of a 17-oxo-15,16-methylene steroid with a sulfoxonium ylide, in particular with dimethysulfoxonium methyl ylide, commonly known as Corey-Chaykovsky reagent. This reagent can be prepared by treatment of trimethylsulfoxonium salts, for example trimethylsulfoxonium chloride or iodide, with a strong base. A preparation procedure is for example disclosed in L.F. Fieser & M. Fieser, Reagents for Organic Synthesis, J. Wiley & Sons, Inc., N.Y., 1967, p. 315.

The resulting spiroepoxides can be subjected to a reaction sequence leading to the desired steroid derivative. In the case of drospirenone, the spiroepoxide is reacted with diethyl malonate to give the corresponding 3-carboxy-spironolactone which, after decarboxylation, provides spironolactone, as illustrated in Scheme 1.

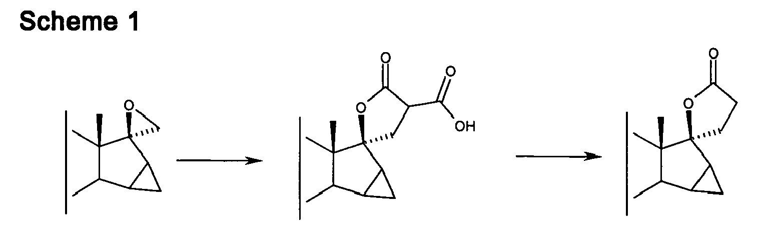

The epoxidation reaction is usually carried out with 1 to 10 equivalents of reagent, preferably from 2 to 5 equivalents, in an anhydrous aprotic organic solvent, preferably selected from dimethylsulfoxide, dimethylformamide, dimethylacetamide, N-methyl-pyrrolidone, sulfolane or ethers and mixtures thereof, such as tetrahydrofuran, dioxane and dimethoxyethane, at a temperature ranging from -10 to 50°C.

The epoxidation reaction is carried out on a steroid substrate which can be subsequently converted to drospirenone according to known methods or as hereinafter disclosed. According to a first preferred embodiment of the invention, the process comprises the reaction sequence illustrated in Scheme 2:

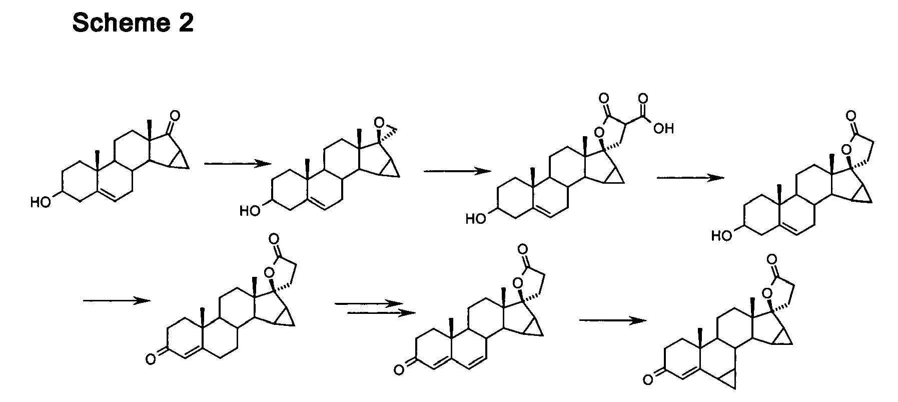

According to a second preferred embodiment, the process comprises the reaction sequence illustrated in Scheme 3:

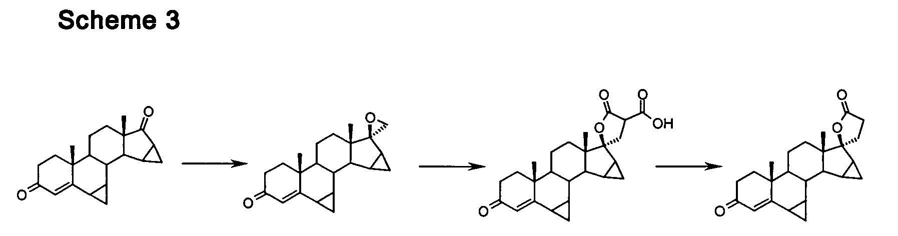

According to a third preferred embodiment, the process comprises the reaction sequence illustrated in Scheme 4.

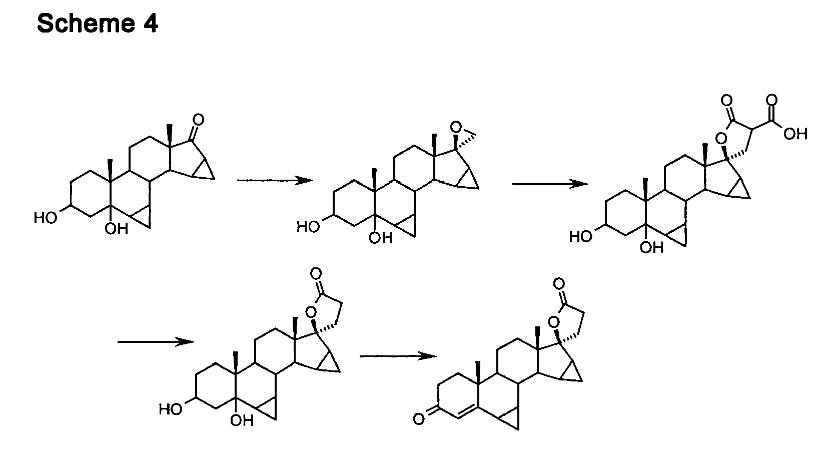

The intermediate 15,16-methylene-17-spiro epoxides are novel and are also object of the present invention. In particular, the following compounds are preferred:

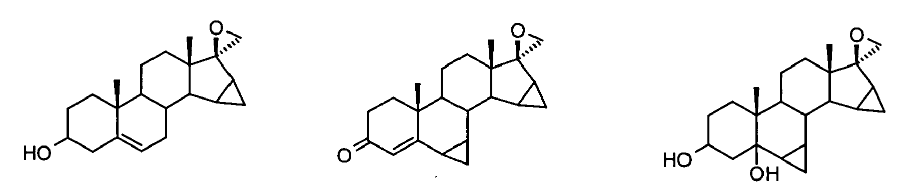

The invention will be now illustrated in greater detail in the following examples.

### EXAMPLES

### Example 1 (process illustrated in Scheme 2)

### Preparation 1: 15β,16β-methylene-17β-spiroxirane androst-5-en-3β-ol

A solution of 183.3 g of trimethylsulfoxonium iodide in 1800 ml dimethylsulfoxide, was added with 33.3 g 60% sodium hydride, stirred at room temperature for 1 hour, then further added with a solution of 50 g 3β-hydroxy-15β,16β-methyleneandrost-5-en-17-one in 600 ml tetrahydrofuran. The reaction mixture was stirred at room temperature for 24 hours, then poured in 6000 g water and ice, stirred at room temperature for 1 more hour and the resulting suspension was filtered and washed with water. After drying, 73.5 g crude 15β,16β-methylene-17β-spiroxiraneandrost-5-en-3β-ol was obtained.

¹H-NMR (CDCl₃): δ (ppm) 0.92 (s, 3H, CH₃); 1.00 (s, 3H, CH₃); 2.82-2.94 (q, 2H, J=5,1 Hz, CH₂); 3,50 (m, 1 H, CH); 5.40 (d, 1 H, J=3,7 Hz,CH).

The crude product was used in the subsequent step without further purification.

### Preparation 2: 3β-hydroxy-15β,16β-methylene-17α-pregn-5-ene-21,17-carbolactone

An ethanol solution of 113.6 g 21% sodium ethoxide and 115.6 g diethyl malonate in 514 ml ethanol was refluxed for 1 hour; after cooling to room temperature, 73.5 g crude 15β,16β-methylene-17β-spiroxiraneandrost-5-en-3β-ol was added. The reaction mixture was refluxed for about 20 hours, concentrated under vacuum and the residue was added with 149 ml ethanol.

This mixture was cooled to 0°C and added with a solution of 73.5 g sodium hydroxide in 1100 ml water, then stirred at room temperature for 20 hours.

The suspension was cooled to 0°C and added with 184 ml 35% hydrochloric acid to adjust the pH from 1 to 2; stirring at 0°C was continued for 3 hours and the suspension was filtered and the resulting solid was washed with water.

After vacuum-drying, the residue (65.3 g) was added with 550 ml dimethylformamide and heated at 130°C for 2-3 hours, then poured in 3427 g water and ice. This mixture was extracted with methylene chloride and the methylene phase was evaporated off under vacuum.

The residue was added with 941 ml water and kept under stirring at room temperature for 12 hours; the suspension was filtered and the resulting solid was washed with water.

The residue obtained after vacuum-drying (57.4 g) was added with 220 ml ethyl ether and kept under stirring for 2 hours at room temperature; the suspension was filtered and washed with ethyl ether.

After vacuum-drying, 36.9 g of 3β-hydroxy-15β,16β-methylene-17α-pregn-5-en-21,17-carbolactone was obtained.

¹H-NMR (CDCl₃): δ (ppm) 0.97 (s, 3H, CH₃); 1.02 (s, 3H, CH₃); 3.50 (m, 1 H, CH); 5.40 (d, 1 H, J=3,7 Hz,CH).

### Preparation 3: 3-oxo-15β,16β-methylene-17α-pregn-4-en-21,17-carbolactone

A mixture of 36.9 g 3β-hydroxy-15β,16β-methylene-17α-pregn-5-en-21,17-carbolactone, 692 ml toluene, 69 ml cyclohexanone and 7.4 g aluminium isopropoxide was refluxed for 3 hours, then cooled to room temperature and added with 634 ml methylene chloride, 288 ml sulfuric acid 1 M and 115 ml water. The resulting phases were separated and the methylene one was washed with 288 ml water, then evaporated under vacuum.

90 g Crude 3-oxo-15β,16β-methylene-17α-pregn-4-en-21,17-carbolactone as an oil was obtained.

¹H-NMR (CDCl₃): δ (ppm) 1.00 (s, 3H, CH₃); 1.20 (s, 3H, CH₃); 5.75 (s, 1 H, CH).

The crude product was used in the subsequent step without further purification.

### Preparation 4: 3-ethoxy-15β,16β-methylene-17α-pregn-3,5-diene-21,17-carbolactone

A solution of 90 g crude 3-oxo-15β,16β-methylene-17α-pregn-4-en-21,17-carbolactone in 74 ml ethanol was added with 56.8 g triethyl orthoformate and 3 g pyridine hydrobromide, then stirred at room temperature for 14 hours. The resulting suspension was cooled at 0°C, added with 2.6 g triethylamine, then stirred at 0°C for 1 hour, filtered and washed with ethanol.

After vacuum drying, 21.4 g 3-ethoxy-15β,16β-methylene-17α-pregn-3,5-diene-21,17-carbolactone was obtained.

¹H-NMR (CDCl₃): δ (ppm) 0.99 (s, 3H, CH₃); 1.00 (s, 3H, CH₃); 5.12 (s, 1 H, CH); 5.24 (m, 1 H, CH).

### Preparation 5: 3-oxo-15β,16β-methylene-17α-pregn-4,6-diene-21,17-carbolactone

A suspension of 20.5 g tetrachloro-p-benzoquinone in 214 ml acetone and 10.7 ml water was added with 21.4 g 3-ethoxy-15β,16β-methylene-17α-pregn-3,5-diene-21,17-carbolactone, then stirred at room temperature for 5 hours, monitoring the reaction by TLC; after this time, the suspension was concentrated under vacuum to obtain a residue which was added with 100 ml of methylene chloride and stirred at 0°C for 1 hour. Thereafter, the insolubles were filtered off, the filtrate was concentrated under vacuum and the residue was purified by column chromatography.

15.9 g 3-Oxo-15β,16β-methylene-17α-pregn-4,6-diene-21,17-carbolactone was obtained.

¹H-NMR (CDCl₃): δ (ppm) 1.07 (s, 3H, CH₃); 1.13 (s, 3H, CH₃); 5.70 (s, 1H, CH); 6.18 (d,d, 1H, J=8.6-2,1 Hz, CH); 6.35 (d,d, 1H, J=8.6-1,1 Hz, CH).

### Preparation 6: Drospirenone

A solution of 11.4 g trimethylsulfoxonium iodide in 223 ml dimethylsulfoxide was added with 2.03 g 60% sodium hydride and stirred at room temperature for 1 hour, then further added with a solution of 15.9 g 3-oxo-15β,16β-methylene-17α-pregn-4,6-diene-21,17-carbolactone in 159 ml dimethylsulfoxide. Stirring was continued at room temperature for 20 hours, then the reaction mixture was poured in 3180 g water and ice; the pH was adjusted to 7 - 7.5 with 3M hydrochloric acid and the solution was extracted with methylene chloride.

The methylene phase was evaporated to a residue, to obtain 16.5 g crude drospirenone, which was subsequently purified by column chromatography to afford 4.7 g of pure product.

### Example 2 (process illustrated in Scheme 3)

### Preparation 1: 15β,16β-methyleneandrost-4-en-3,17-dione

A suspension of 30 g 3β-hydroxy-15β,16β-methyleneandrost-5-en-17-one in 500 ml toluene was added with 50 ml cyclohexanone and 7.56 g aluminium isopropoxide, stirring under reflux for 1 hour. The mixture was cooled to room temperature and added with 500 ml methylene chloride and 300 ml 1 M sulfuric acid; the phases were separated and the organic one was washed with 300 ml water, then concentrated under vacuum.

The residue was taken up with 50 ml cyclohexane, whereby the title product started to crystallize; the suspension was cooled at 0°/5°C for 2 hours, then filtered and washed with cyclohexane. After drying, 22.3 g 15β,16β-methyleneandrost-4-en-3,17-dione was obtained.

¹H-NMR (CDCl₃): δ (ppm) 0.99 (s, 3H, CH₃); 1.19 (s, 3H, CH₃); 5.74 (s, 1 H, CH).

### Preparation 2: 15β,16β-methyleneandrost-4,6-diene-3,17-dione

A mixture of 15 g 15β,16β-methyleneandrost-4-en-3,17-dione, 300 ml ter-butanol and 18.5 g tetrachloro-p-benzoquinone was refluxed for 3 hours. After cooling at 30°/40°C, the insolubles were filtered off and the mother liquor was concentrated under vacuum. The residue was taken up with 300 ml methylene chloride and 150 ml water; the suspension was filtered and the liquid phases were separated; the organic one was washed with 2 x 150 ml water; the phases were separated and the organic one was added with a 5% sodium hydroxide solution (450 ml); the suspension was filtered through Celite and the liquid phases were separated. The organic phase was washed with a 5% sodium hydroxide solution (2 x 150 ml); the phases were separated and the organic one was washed with a 4% sodium chloride solution (3 x 150 ml). The organic phase was concentrated under vacuum and the residue was taken up with 50 ml tert-butyl methyl ether, cooled to 0°/5°C for 30 minutes and the resulting suspension was filtered.

After vacuum drying, 8.7 g 15β,16β-methyleneandrost-4,6-diene-3,17-dione was obtained.

¹H-NMR analysis (CDCl₃): δ (ppm) 1.04 (s, 3H, CH₃); 1.12 (s, 3H, CH₃); 5.70 (s, 1 H, CH); 6.22 (d,d, 1 H, J=8,6 - 2,1 HZ, CH); 6.37 (d,d, 1 H, J=8.6 - 1,1 HZ, CH).

### Preparation 3: 6β,7β,15β,16β-Dimethylene-17β-spiroxiraneandrost-4-en-3-one

A solution of 7.44 g trimethylsulfoxonium iodide in 72 ml dimethylsulfoxide was added with 1.35 g 60% sodium hydride, then stirred at room temperature for 1 hour and further added with a solution of 2 g 15β,16β-methyleneandrost-4,6-diene-3,17-dione in 24 ml tetrahydrofuran. Stirring was continued at room temperature for 20 hours, monitoring the reaction by TLC; when the reaction was complete, the mixture was poured in 240 g water and ice and diluted with 200 ml methylene chloride; the phases were separated and the aqueous one was extracted with 50 ml methylene chloride, then separated again. The organic phases were pooled and washed with 100 ml water, then concentrated to dryness to obtain 1.9 g crude 6β,7β,15β,16β-dimethylene-17β-spiroxiraneandrost-4-en-3-one.

¹H-NMR (CDCl₃): δ (ppm) 0.93 (s, 3H, CH₃); 1.08 (s, 3H, CH₃); 5.70 (s, 1 H, CH); 2.87-2.96 (q, 2H, J=5 HZ, CH₂); 6.02 (s, 1 H, CH).

The crude product was used in the subsequent step without further purification.

### Preparation 4: Drospirenone

An ethanol solution of 3.5 g 21% sodium ethoxide and 3.75 g diethyl malonate in 15 ml ethanol was refluxed for 1 hour; after cooling at room temperature, 1.8 g of crude 6*β*,7β,15β,16β-dimethylene-17β-spiroxirananedrost-4-en-3-one was added. The reaction mixture was refluxed for 3 hours, monitoring the reaction by TLC; when the reaction was complete, the mixture was concentrated under vacuum to a residue and added with 5 ml ethanol.

The mixture was cooled at 0°C and added with a solution of 2.4 g sodium hydroxide in 40 ml water, stirring at room temperature for 20 hours.

The solution was cooled at 0°C and the pH was adjusted to 1 - 2 with 35% hydrochloric acid, stirring at 0°C for 3 hours; the resulting suspension was filtered and washed with water.

After vacuum drying, a residue was obtained (1.8 g), which was added with 10 ml dimethylformamide and heated at 130°C for 2.5 hours, monitoring the reaction by TLC; the reaction mixture was poured in 50 g water and ice and extracted with methylene chloride. The methylene phase was concentrated under vacuum to give a residue which, after purification by column chromatography, provided 0.5 g drospirenone.

## Claims

1. A process for the epoxidation of 17-oxo-15,16-methylene steroids comprising the treatment of a 17-oxo-15,16-methylene-steroid with a sulfoxonium ylide.

2. Process as claimed in claim 1 wherein the sulfoxonium ylide is dimethylsulfoxonium methyl ylide.

3. Process as claimed in claim 1 or 2 in which the 17-oxo-15,16-methylene steroid is a precursor of drospirenone (I)

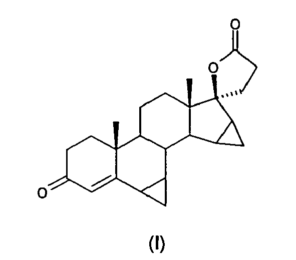

4. Process as claimed in claim 3 in which the drospirenone precursor is selected from:

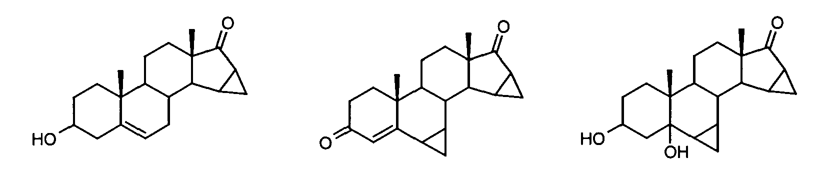

5. Process as claimed in claim 3 or 4 in which the product of the epoxidation reaction is reacted with diethyl malonate and subjected to decarboxylation to give drospirenone.

6. Process according to any one of claims 2-5 in which the epoxidation is carried out using 1 to 10 equivalents of a trimethylsulfoxonium salt with respect to the steroid.

7. Process as claimed in claim 6 in which from 2 to 5 equivalents of trimethylsulfoxonium salt with respect to the steroid were used.

8. Process according to any one of claims 1-7 in which the epoxidation is carried out in an anhydrous organic aprotic solvent.

9. Process as claimed in claim 8 in which the solvent is selected from: dimethylsulfoxide, sulfolane, dimethylformamide, dimethylacetamide, N-methyl-pyrrolidone, ethers and mixtures thereof.

10. Process according to any one of claims from 3 to 9 as illustrated in the following scheme:

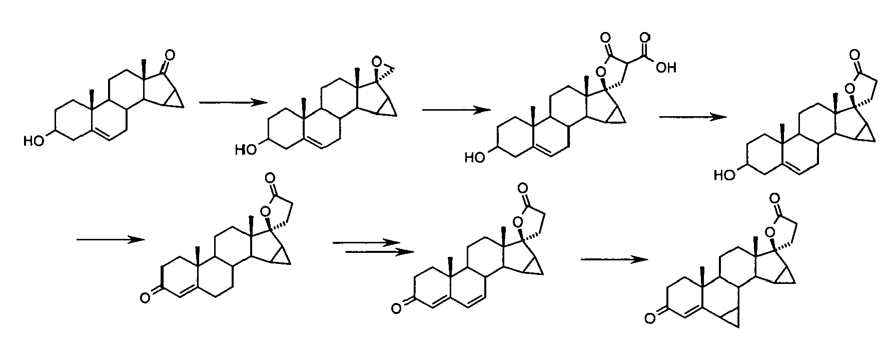

11. Process according to any one of claims from 3 to 10 as illustrated in the following scheme:

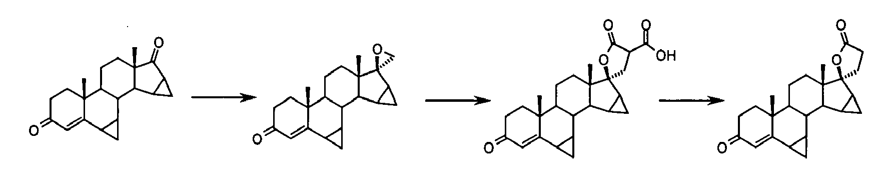

12. Process according to any one of claims from 3 to 11 as illustrated in the following scheme:

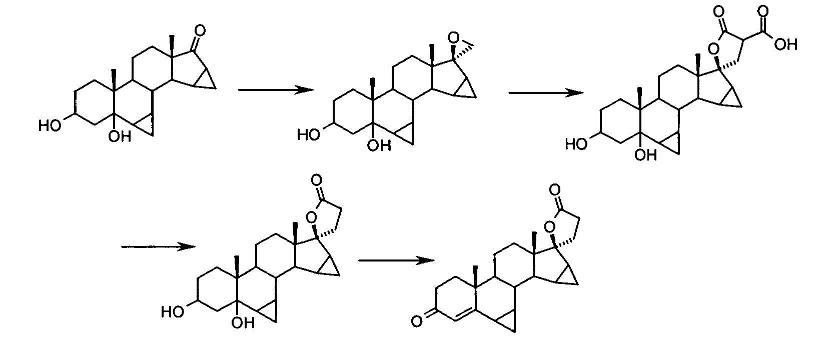

13. A compound selected from:
